# EUROPEAN PATENT APPLICATION

(11) **EP 4 169 907 A1**
(43) Date of publication of application: **26.04.2023**
(21) Application number: 21306479.3
(22) Date of filing: 25.10.2021
(51) Int. Cl.: C07D 231/16, C06B 25/34

(54) **NITRAMINE PYRAZOLE ENERGETIC COMPOUNDS**

(71) Applicant: Eurenco, 84700 Sorgues (FR)
(72) Inventor: Klapötke, Thomas, 81377 Munich (DE); Stierstorfer, Joerg, 81377 Munich (DE); Reinhardt, Elena, 81377 Munich (DE)
(74) Representative: Cabinet Beau de Loménie

(57) **Abstract**

The invention relates to a compound of formula (I): 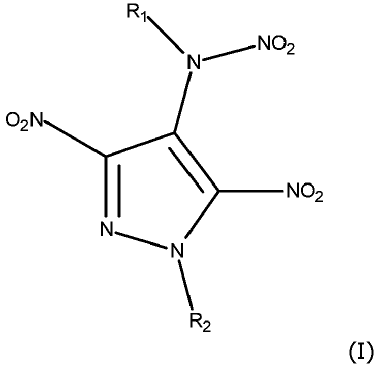 wherein R₁ and R₂ are as defined in the description.

The invention also relates to a composition comprising a compound of formula (I).

## Description

### Field of the invention

The present invention is in the field of energetic materials, and more specifically relates to energetic compounds, to an energetic composition comprising said compounds, and to a process for the synthesis of said compounds.

### Background of the invention

In the last decades of energetic materials research, the search for environmental benign alternatives for commonly used compounds is one of the main objectives [1]. Heavy metal-free primary explosives and pyrotechnics, non-toxic secondary explosives and halogen-free oxidizers are still under investigation. "Green" high energy dense oxidizers (HEDOs) are generally developed to replace ammonium perchlorate (AP), which is employed as standard oxidizer in composite propellant mixtures for rocket and missile engines [1]. Ammonium perchlorate has numerous advantages such as its outstanding low price, great availability and favourable oxygen balance [1]. Nonetheless the perchlorate ion is a hazardous substance and is registered under the REACH Regulation [2]. Additionally upon combustion, large amounts of chlorinated products are formed and released in the atmosphere. These exhaust gases directly harm the environment and can be detected easily, leading to tactical disadvantages [1]. Several potential halogen-free alternatives have been synthesized over the last decades. Ionic compounds such as ammonium dinitramide and ammonium nitrate appear to be potent replacements [3-4]. Covalent structures generally utilize specific oxygen-rich moieties. Some promising alternatives are based on the trinitroethyl moiety, such as trinitroethyl nitrocarbamate, bis(trinitroethyl)oxalate and 2,2,2-trinitroethyl formate [5-7].

There is therefore an on-going need to find alternative energetic materials. The present invention aims at providing such materials.

### Summary of the invention

In one aspect, the invention relates to a compound of formula (I): 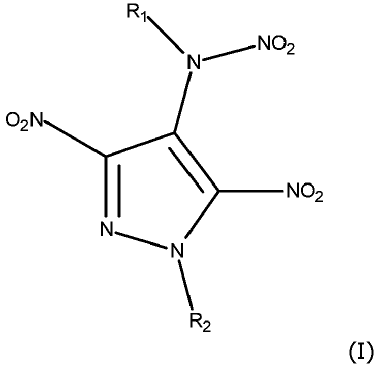 wherein R₁ and R₂ each independently represent H or a (C₁-C₄)alkyl, provided that when R₁ is methyl then R₂ is not H.

In another aspect, the invention relates to a process for synthesizing the compound of formula (I).

In another aspect, the invention relates to an energetic composition comprising a compound of formula (I).

### Description of the figures

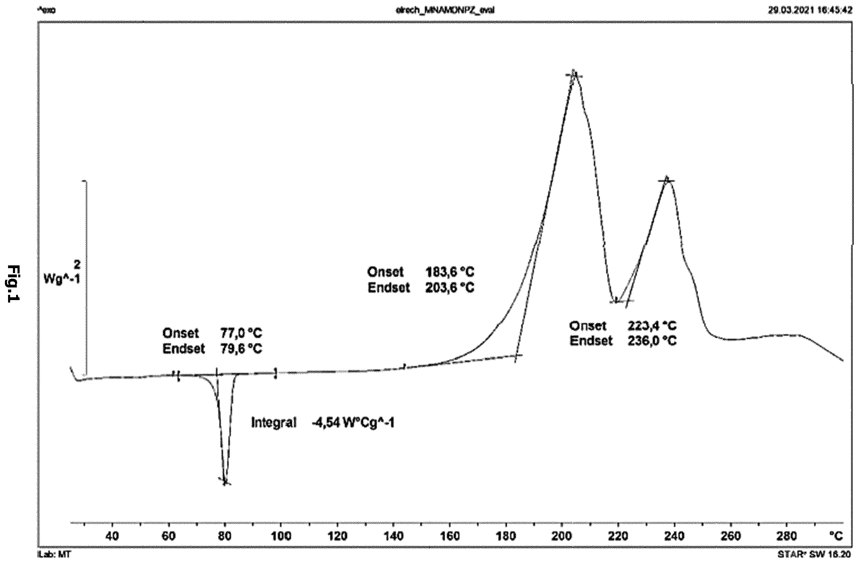

Figure 1 represents a DSC curve of a representative compound of formula (I).

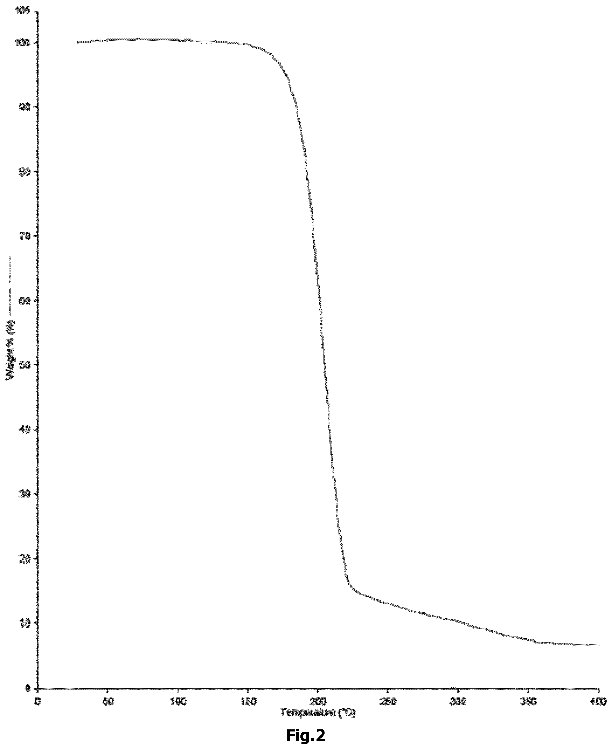

Figure 2 represents a TGA curve of a representative compound of formula (I).

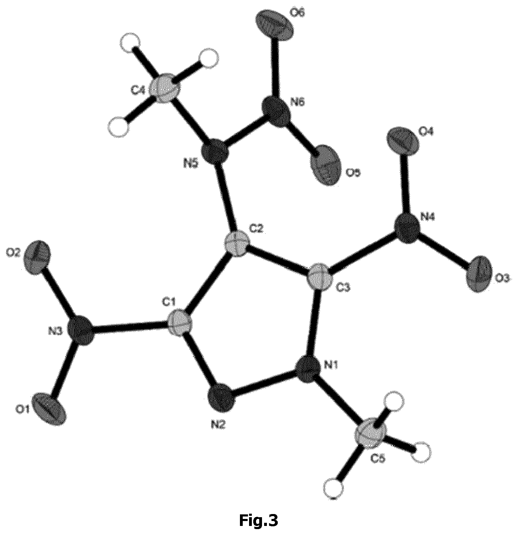

Figure 3 represents the crystal structure of a representative compound of formula (I).

### Detailed description of the invention

In one aspect, the invention relates to a compound of formula (I): 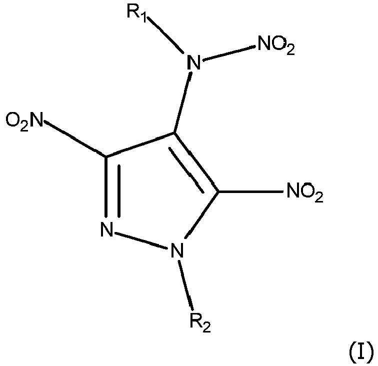 wherein R₁ and R₂ each independently represent H or a (C₁-C₄)alkyl, provided that when R₁ is methyl then R₂ is not H.

In the context of the present invention, the "(C₁-C₄)alkyl" includes methyl, ethyl, n-propyl, i-propyl, n-butyl, sec-butyl (1-methylpropyl), isobutyl (2-methylpropyl) and tert-butyl.

In some embodiments, R₁ and R₂ are each a (C₁-C₄)alkyl, and R₁ and R₂ can be identical or different.

In some embodiments the compound of formula (I) is 3,5-dinitro-4-methylnitramino-1-methyl-1H-pyrazole (i.e. a compound of formula (I) where R₁ and R₂ are each a methyl group).

In one aspect, the invention relates to a process for synthesizing a compound of formula (I), which comprises:
a) nitration of a compound of formula (II): 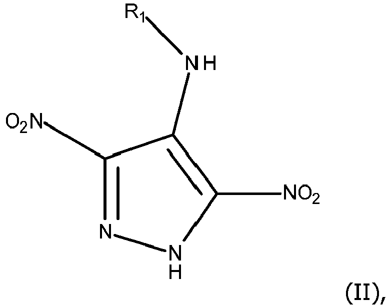 wherein R₁ is H or a (C₁-C₄)alkyl, to obtain a compound of formula (I) where R₂ is H: 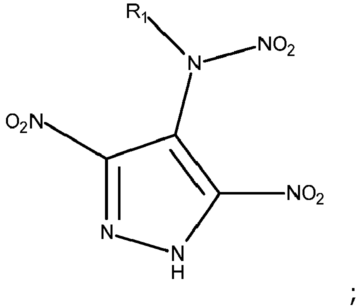 and
b) optionally, alkylation of the compound obtained in step a).

The compounds of formula (II) can be obtained by nucleophilic substitution of a compound of formula (III) with the appropriate amine:

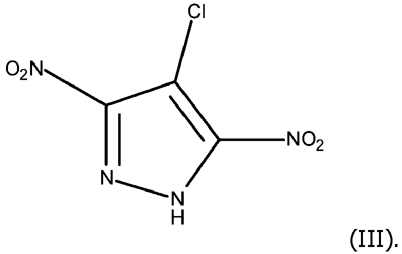

The compound of formula (III) can be obtained by acid nitration of 4-chloropyrazole, which itself can be obtained from pyrazole.

The compounds of formula (I) display good energetic performances and safety parameters and can therefore be used as energetic materials, notably as an alternative to TNT.

Thus according to another aspect the invention relates to an energetic composition comprising a compound of formula (I) and at least one compound selected from an additive and a metal.

In some embodiments the amount of compound of formula (I) in the energetic composition is at least 70 wt%, such as for example at least 75 wt%, at least 80 wt%, at least 85 wt% or at least 90 wt%, of the weight of the energetic composition.

In some embodiments the energetic composition comprises an additive. In some embodiments the additive includes a processing agent, a plasticizer, an anti-cracking agent and mixtures thereof.

In some embodiments the additive represents up to 10 wt%, for example up to 5 wt%, of the weight of the energetic composition.

In some embodiments the energetic composition comprises a metal such as, for example, Al or Mg.

In some embodiments the metal represents up to 30 wt%, for example up to 20 wt%, up to 10 wt% or up to 5 wt%, of the weight of the energetic composition.

In some embodiments, the energetic composition further comprises at least one energetic material in addition to the compound of formula (I). In some embodiments the at least one additional energetic material includes trinitrotoluene (TNT), hexogen (RDX), octogen (HMX), hexanitrohexaazaisowurtzitane (CL20), nitroguanidine (NGU), ethylene dinitramine (EDNA), N-guanylurea dinitramide (FOX 12 (GUDN)), 1,1-diamino-2,2-dinitroethylene (FOX 7 (DADE)), 5,5'-azotetrazolate of bis(triaminoguanidinium) (TAGZT), 5,5' azotetrazolate of dihydrazinium (DHDZT), 5,5'-bis(tetrazolyl) hydrazine (HBT), bis(2,2-dinitropropyl)nitramine (BDNPN), a nitropyrazole such as for example 3,4,5-trinitropyrazole (3,4,5-TNP), dihydroxylammonium 5-5'-bistetrazole-1,1'-diolate (TKX-50), and mixtures thereof. In such embodiments, the amount of energetic materials (i.e., the compound of formula (I) and the at least one other energetic material) in the energetic composition is at least 70 wt%, such as for example at least 75 wt%, at least 80 wt%, at least 85 wt% or at least 90 wt% of the weight of the energetic composition. In other words part of the compound of formula (I) can be replaced by the at least one other energetic material.

The invention will be better understood in the light of the examples which are given by way of illustration.

### Examples

NMR spectra were recorded with JEOL Eclipse and Bruker TR 400 MHz spectrometers at 25 °C. Chemical shifts were determined with respect to external standards Me₄Si (¹H, 399.8 MHz); (¹³C, 100.5 MHz); MeNO₂ (¹⁴N/¹⁵N, 28.9/40.7 MHz). Elemental analyses (EA) were obtained with a Vario EL Elemental Analyzer.

The sensitivity data were acquired by measurements with a BAM drop hammer and a BAM friction tester (BAM stands for "Bundesanstalt für Materialforschung und -prüfung"). Melting and decomposition points were determined using a Differential Thermal Analyzer from the company OZMResearch (model DTA 552-Ex) at a heating rate of 5°C/min. Measurements were performed in open glass vessels against a reference material up to 400°C.

The crystal structure data were obtained using an Oxford Xcalibur CCD Diffraktometer with a KappaCCD detector at low temperature (173 K, 123 K). Mο-Kα radiation (λ = 0.71073 Å) was delivered by a Spellman generator (voltage 50 kV, current 40 mA). Data collection and reduction were performed using the CRYSALIS CCD [8] and CRYSALIS RED [9] software, respectively. The structures were solved by SIR92/SIR97 [10] (direct methods) and refined using the SHELX-97 [11-12] software, both implemented in the program package WinGX22 [13]. Finally, all structures were checked using the PLATON software [14]. Structures displayed with ORTEP plots are drawn with thermal ellipsoids at 50 % probability level.

The theoretical calculations were achieved by using the GAUSSIAN16 program package [15] and were visualized by using GAUSSVIEW 6.0.16 [16]. Optimizations and frequency analyses were performed at the B3LYP level of theory (Becke's B3 three parameter hybrid functional by using the LYP correlation functional) with a cc-pVDZ basis set. After correcting the optimized structures with zero-point vibrational energies, the enthalpies and free energies were calculated on the CBS-4M (complete basis set) level of theory [17]. The detonation parameters were obtained by using the EXPLO5 (V6.05) program package [18-19].

### Abbreviations

Ac₂O: acetic anhydride
EtOAc: ethyl acetate
TFA: trifluoroacetic acid

### Example 1: 4-Chloro-1H-pyrazole

Sodium hypochlorite solution (350 mL, 13% active Cl₂, 0.7 mL/mmol) was slowly added to a solution of 1*H*-pyrazole (34.00 g, 0.50 mol) in H₂O (100 mL) while keeping the temperature below 35°C. The solution was stirred for 1h at room temperature, brought to pH=9 using HCl (37 %) and cooled with ice. The precipitate was filtered off to give 4-chloropyrazole (35.40 g, 0.35 mol) in 69% yield as a colorless powder.

**¹H NMR** (400 MHz, DMSO-d*6*, 25 °C) δ (ppm) = 7.75 (s, 1H), 13.15 (s, 2H); **¹³C{¹H} NMR** (101 MHz, DMSO-d*6*, 25 °C) δ (ppm) = 108.0, 131,6; **EA** (C₃H₃ClN₂, 102.52 g/mol) calc. (found): C 35.15 (35.02), H 2.95 (2.80), N 27.32 (27.27) %; **MS** (EI+) *m*/*z*: 101.87 [C₃H₃ClN₂]; **IR** (ATR, 25 °C), *ṽ*(rel. int.): 3158 (s), 3086 (s), 2999 (s), 2935 (s), 2884 (s), 2821 (s), 1643 (m), 1559 (m), 1504 (m), 1379 (s), 1340 (s), 1288 (m), 1197 (s), 1171 (m), 1141 (m), 1071 (w), 1034 (s), 1007 (m), 970 (s), 954 (s), 938 (vs), 843 (vs), 833 (vs), 808 (s), 792 (vs), 644 (m), 604 (vs), 533 (m), 473 (s) cm⁻¹.

### Example 2: 4-Chloro-3,5-dinitro-1H-pyrazole

4-Chloropyrazole (15.70 g, 153 mmol, 1.00 eq) was slowly added to H₂SO₄ (96%, 191 mL, 1.25 mL/mmol) at 0°C. Then HNO₃ (100%, 20.2 mL. 484 mmol, 0.13 mL/mmol, 3.2 eq.) was added dropwise to the mixture at 0°C. The reaction mixture was heated at 100°C for 7h. Afterwards the mixture was allowed to cool down to room temperature and then poured onto ice water (2 L). The aqueous solution was extracted with EtOAc (3 × 300 mL), the combined organic extracts were dried over anhydrous MgSO₄, filtered and concentrated under reduced pressure. Crystallization from EtOAc gave 4-chloro-3,5-dinitropyrazole (19.30 g, 100 mmol) in 66% yield as yellow blocks.

**¹H NMR** (400 MHz, DMSO-*d*6, 25 °C): δ (ppm) = 10.82 (s); **¹³C{¹H} NMR** (101 MHz, DMSO-d6, 25 °C): δ (ppm) = 148.4, 103.5; **¹⁴N{¹H} NMR** (29 MHz, DMSO-d*6*, 25 °C): δ (ppm) = -27; **EA** (C₃HClN₄O₄, 192.52 g/mol) calc. (found): C 18.72 (19.01), H 0.52 (0.73), N 29.10 (29.04) %. **MS** (EI+) *m*/*z*: 191.89 [C₃HClN₄O₄]; **IR** (ATR, 25 °C), *ṽ*(rel. int.): 3242 (w), 1567 (m), 1510 (m), 1420 (m), 1321 (m), 1209 (m), 1114 (m), 1001 (m), 837 (m), 777 (m), 760 (m), 681 (m), 615 (m), 590 (m), 511 (m) cm⁻¹.

### Example 3: N-methyl-3,5-dinitro-1H-pyrazol-4-amine monohydrate

To a solution of CH₃NH₂ (40 wt.% in H₂O, 135 mL, 1.57 mol, 1.6 mL/mmol) was added 4-chloro-3,5-dinitropyrazole (15.86 g, 82.38 mmol). The mixture was heated at 140°C for 6h in a steel-autoclave. Afterwards the steel-autoclave was allowed to cool up to room temperature, the reaction mixture was acidified with H₂SO₄ (96%) to p*H* = 1-2. The suspension was filtered and washed with ice-cold H₂O to give 3,5-dinitro-4-methylaminopyrazole as monohydrate (15.53 g, 75.71 mmol) in 92% yield as a yellow powder.

**¹H NMR** (400 MHz, DMSO-*d*6, 25 °C): δ (ppm) = 7.88 (s, 1H), 4.11 (s, 3H); **¹³C{¹H} NMR** (101 MHz, DMSO-*d*6, 25 °C): δ (ppm) = 139.0, 129.7, 33.4; **¹⁴N{¹H} NMR** (29 MHz, DMSO-*d*6, 25 °C): δ (ppm) = -28; **EA** (C₄H₇N₅O₅, 205.13 g/mol) calc. (found): C 23.42 (24.68), H 3.44 (3.02), N 34.14 (35.96) %. **MS** (EI+) *m*/*z*: 187.03 [C₄H₅N₅O₄].

### Example 4: N-methyl-3,5-dinitro-1H-pyrazol-4-amine monohydrate

To a solution of 4-chloro-3,5-dinitropyrazole (3.50 g, 18.18 mmol, 1.0 eq.) in H₂O (20 mL) CH₃NH₂ (40 wt.% in H₂O, 18 mL, 209 mmol, 1 mL/mmol, 11.5 eq.) was added. The mixture was heated at 105°C overnight. Afterwards the reaction was allowed to cool up to room temperature and was acidified with HCl (37%) to p*H* = 1-2. The suspension was filtered, washed with ice-cold H₂O and dried at 50 °C to give 3,5-dinitro-4-methylaminopyrazole (2.98 g, 15.42 mmol) in 88% yield as an orange powder.

**¹H NMR** (400 MHz, DMSO-*d*6, 25 °C): δ (ppm) = 7.59 (s, 1H), 302 (s, 3H); **¹³C{¹H} NMR** (101 MHz, DMSO-*d*6, 25 °C): δ (ppm) = 139.9, 130.1, 33.3; **EA** (C₄H₅N₅O₄, 187.12 g/mol) calc. (found): C 25.68 (25.73), H 2.69 (2.96), N 37.43 (37.54) %. **MS** (EI+) *m*/*z*: 187.03 [C₄H₅N₅O₄].

### Example 5: 3,5-dinitro-4-methylnitramino-1H-pyrazole

3,5-Dinitro-4-methylaminopyrazole monohydrate (9.50 g, 46.31 mmol, 1.0 eq.) was added portion wise to TFA (100 mL, 2.2 mL/mmol) at 0°C. Then HNO₃ (100%, 33.0 mL, 79.08 mmol, 0.71 mL/mmol, 1.7 eq.) was added dropwise to the mixture. Afterwards Ac₂O (10.0 mL, 105.7 mmol, 0.22 mL/mmol, 2.3 eq.) was added slowly and the mixture was stirred at 0°C for 3h. The reaction mixture was poured onto ice and extracted with EtOAc (3 × 200 mL). The combined organic extracts were dried over MgSO₄, filtered and concentrated under reduced pressure. Crystallization from EtOAc gave 3,5-dinitro-4-methylnitraminopyrazole (10.30 g, 44.37 mmol) in 96% yield in the form of yellow crystals.

**¹H NMR** (400 MHz, DMSO-d6, 25 °C): δ (ppm) = 9.62 (s, 1H), 3.63 (s, 3H); **¹³C{¹H} NMR** (101 MHz, DMSO-d6, 25 °C): δ (ppm) = 147.5, 111.4, 40.5; **¹⁴N{¹H} NMR** (29 MHz, DMSO-d6, 25 °C): δ (ppm) = -24, -31; **EA** (C₄H₄N₆O₆, 232.11 g/mol) calc. (found): C 20.70 (20.31), H 1.74 (1.81), N 36.21 (35.67) %. **HRMS** (EI+) *m*/*z*: 187.0335 [C₄H₅N₅O₄] (M-NO₂).

### Example 6: 3,5-dinitro-4-methylnitramino-1-methyl-1H-pyrazole

3,5-Dinitro-4-methylnitramino-1H-pyrazole (1.50 g, 6.46 mmol, 1.00 eq.) was added to a solution of sodium bicarbonate (1.00 g, 11.9 mmol, 1.84 eq.) in water (20 mL). After stirring for 10 min, dimethyl sulfate (0.68 mL, 7.17 mmol, 1.10 eq.) was added. The reaction mixture was stirred at room temperature for 1.5 h. The precipitate formed was filtered off and dried in the air to give the title compound as an off-white powder (1.10 g, 4.20 mmol, 69 %).

**¹H NMR** (400 MHz, DMSO-*d*6, 25 °C, ppm) δ = 4.35 (s, 3H), 3.68 (s, 3H). **¹³C NMR** (101 MHz, DMSO-*d*6, 25 °C, ppm) δ = 145.2, 140.3, 113.0, 43.2, 40.2. **¹⁴N NMR** (29 MHz, DMSO-*d*6, 25 °C, ppm) δ = - 28, - 32. **¹⁵N NMR** (41 MHz, DMSO-*d*6, 25 °C, ppm) δ = - 27, - 33, - 77, - 183, - 222. **EA** (C₅H₆N₆O₆): calc.: C 24.40, H 2.46, N 34.14 %, found: C 24.18, H 2.65, N 34.04 %. **HRMS** (ESI⁻): 291.03335 ([C₅H₆N₆O₆] + [HCOO⁻]).

### Example 7 and comparative example 1: characterization of 3,5-dinitro-4-methylnitramino-1-methyl-1H-pyrazole and of TNT

The energetic properties of the compounds have been determined and are presented in table 1.

**Table 1**

| Formula | C₅H₆N₆O₆ | TNT |
|---|---|---|
| Density [g cm⁻³] | 1.648 | 1.654 |
| IS [J] | 15 | 15 |
| FS [N] | > 360 | > 360 |
| Ω [%] | -45 | -74 |
| Tₘₑₗₜ (°C) | 77 | 80 |
| T_{dec} (°C) | 184 | 295 |
| Δ_{f}H° [kJ mol⁻¹] | 135.2 | -59.3 |
| **EXLPO5 6.05.02 values** | | |
| *-*Δ*_{E}U*° [kJ kg⁻¹] | 5147 | 4427 |
| *p_{C-J}* [GPa] | 24.1 | 19.4 |
| *D*[m s⁻¹] | 7720 | 6824 |

| | | |
|---|---|---|
| IS: impact sensitivity / FS: friction sensitivity / Tmelt: melting temperature / Tdec: decomposition temperature / Ω: oxygen balance / Δ_{f}H : enthalpy of formation / -Δ_{E}U: enthalpy of internal energy / p_{C-J}: pressure of Chapman Jouguet / D: detonation velocity | | |

It can be seen from table 1 that 3,5-dinitro-4-methylnitramino-1-methyl-1H-pyrazole has a melting temperature (77°C) which is comparable to that of TNT (80°C) and has a detonation velocity (7720 m.s⁻¹) which is higher than that of TNT (6824 m.s⁻¹). It also has a density comparable to that of TNT (1.648 vs 1.654 g.cm⁻³).

### Example 8: characterization of the crystal structure of 3,5-dinitro-4-methylnitramino-1-methyl-1H-pyrazole

3,5-dinitro-4-methylnitramino-1-methyl-1H-pyrazole crystallized in the space group P2₁/c with a density of 1.696 g/cm³ (@184 K) from water/acetonitrile. The crystal structure and data were obtained as described in the section "Materials and Methods". Said data are collated in the table below.

**Table 2**

| formula | C₆H₈N₆O₁₂ |
|---|---|
| *T*[K] | 102 |
| λ [Å] | 0.71073 |
| crystal system | Monoclinic |
| space group | P21/c (No. 14) |
| size [mm] | 0.21 x 0.39 x 1.00 |
| color habit | colorless / block |
| *a*[Å] | 8.2862(7) |
| *b*[Å] | 11.6872(8) |
| *c*[Å] | 10.5905(9) |
| α[°] | 90 |
| *β*[°] | 109.912(10) |
| *γ*[°] | 90 |
| *V*[Å³] | 964.30(15) |
| *Z* | 4 |
| ρ_{calc.} [g cm⁻³] | 1.696 |
| *µ*[mm⁻¹] | 0.155 |
| F(000) | 504 |
| θ Min-Max [°] | 2.6, 26.4 |
| dataset | -10:9; -14:14; -13:13 |
| reflections collected | 9162 |
| independent reflections | 1973 |
| parameters | 178 |
| GooF | 1.05 |
| R₁/wR₂ (obs) | 0.0333/0.0787 |
| R₁/wR₂ (all data) | 0.0417/0.0832 |
| residual density [e Å⁻³] | -0.19, 0.25 |

### Example 9: compatibility with other energetic materials

The compound of example 6, i.e. 3,5-dinitro-4-methylnitramino-1-methyl-1H-pyrazole, was found to be compatible with other energetic materials such as HMX and RDX, as determined by DSC.

### References

[1] T. M. Klapötke, Chemistry of High-Energy Materials, 5th ed., Walter de Gruyter, Berlin, 2019
[2] https://echa.europa.eu/substance-information/-/substanceinfo/100.028.647 (accessed 07.2021)
[3] H. F. R. Schoeyer, A. J. Schnorhk, P. A. O. G. Korting, P. J. van Lit, J. M. Mul, G. M. H. J. L. Gadiot, J. J. Meulenbrugge, J. Propul. Power 1995, 11, 856-869
[4] J. C. Bottaro, P. EI. Penwell, R. J. Schmitt, J. Am. Chem. Soc. 1997, 119, 9405-9410
[5] Q. J. Axthammer, T. M. Klapötke, B. Krumm, R. Moll, S. F. Rest, Z. Anorg. Allg. Chem. 2014, 640, 76-83
[6] Q. J. Axthammer, B. Krumm, T. M. Klapötke, J. Org. Chem. 2015, 80, 6329-6335
[7] T. M. Klapötke, B. Krumm, R. Scharf, Eur. J. Inorg. Chem. 2016, 3086-3093
[8] 1.171.35.11 ed., Oxford Diffraction Ltd., Abingdon, Oxford (U.K.), **2011**
[9] 1.171.35.11 ed., Oxford Diffraction Ltd., Abingdon, Oxford (U.K.), **2011**
[10] A. Altomare, M. C. Burla, M. Camalli, G. L. Cascarano, C. Giacovazzo, A. Gualiardi, A. G. G. Moliterni, G. Polidori, R. Spagna, J. Appl. Crystallogr. 1999, 32, 155-119
[11] G. M. Sheldrick, Programs for Crystal Structure Determination, University of Göttingen, Germany, 1997
[12] G. M. Sheldrick, Acta Crystallogr. 2008, 64A, 112-122
[13] L. Farrugia, J. Appl. Crystallogr. 1999, 32, 837-838
[14] A. L. Spek, Acta Crystallogr. 2009, 65D, 148-155
[15] http://gaussian.com/
[16] R. D. Dennington, T. A. Keith, J. M. Millam, GaussView, Ver. 6.0.16 ed., Semichem Inc., Shawnee Mission, KS, 2016
[17] J. A. Montgomery, M. J. Frisch, J. W. Ochterski, G. A. Petersson, J. Chem. Phys. 2000, 112, 6532-6542
M. Sućeska, EXPLO5 V.6.03, Zagreb, 2015
M. Sućeska, Propellants, Explos. Pyrotech. 1991, 16, 197-202

## Claims

1. A compound of formula (I): 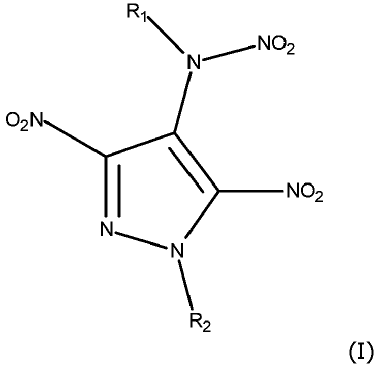 wherein R₁ and R₂ are each independently H or a (C₁-C₄)alkyl, provided that when R₁ is methyl then R₂ is not H.

2. The compound of claim 1, wherein R₁ and R₂ are each a (C₁-C₄)alkyl.

3. The compound of claim 2, wherein R₁ and R₂ are identical or different.

4. The compound of claim 1, which is 3,5-dinitro-4-methylnitramino-1-methyl-1H-pyrazole.

5. An energetic composition comprising a compound of formula (I) as defined in one of claims 1 to 4, and at least one compound selected from an additive and a metal.

6. The energetic composition of claim 5, which comprises at least 75 wt% of compound of formula (I), based on the weight of the energetic composition.

7. The energetic composition of claim 5, which further comprises at least one additional energetic material.

8. The energetic composition of claim 7, wherein 3,5-dinitro-4-methylnitramino-1-methyl-1h-pyrazole and the at least one additional energetic material make up at least 75 wt% of the composition.

9. A process for synthesizing a compound of formula (I) as defined in claim 1, which comprises:
a) nitration of a compound of formula (II): 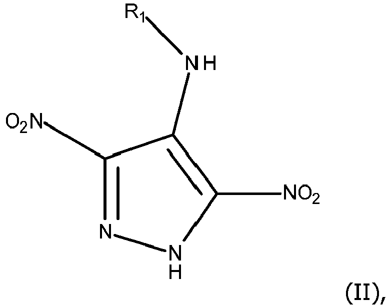 wherein R₁ is a (C₁-C₄)alkyl, to obtain a compound of formula (I) where R₂ is H: 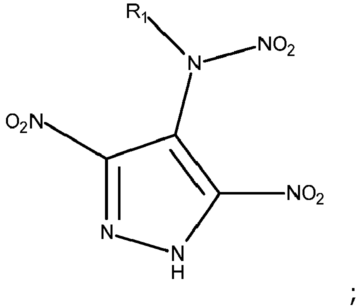 and
b) optionally, alkylation of the compound obtained in step a).
